# EUROPEAN PATENT APPLICATION

(11) **EP 4 613 308 A1**
(43) Date of publication of application: **10.09.2025**
(21) Application number: 23886216.3
(22) Date of filing: 31.10.2023
(51) Int. Cl.: A61M 15/00, A61M 11/00

(54) **INHALER**

(30) Priority: 02.11.2022 KR 20220144267
(71) Applicant: KT&G Corporation, Daedeok-gu Daejeon 34337 (KR)
(72) Inventor: KIM, Moonwon, Daejeon 34128 (KR); JUNG, Yongmi, Daejeon 34128 (KR); SHIN, Junwon, Daejeon 34128 (KR)
(74) Representative: Ter Meer Steinmeister & Partner
(86) International application number: PCT/KR2023/017134
(87) International publication number: WO 2024/096520

(57) **Abstract**

An inhaler according to an embodiment comprises: a main body comprising a first surface and a second surface facing the first surface and provided with a mouthpiece, and having formed therein a channel in a longitudinal direction, the channel facing the second surface on the first surface; a cartridge which is coupled to the first surface of the main body to be in communication with the channel and contains inhalable powder; and a plurality of baffles arranged in the channel, wherein, when inhaling force is applied to the mouthpiece, the baffles can restrict the particle size of the inhalable powder that is shifted to the mouthpiece.

## Description

### TECHNICAL FIELD

An inhaler is disclosed.

### BACKGROUND ART

In general, an inhaler is a device used to inhale a composition such as a drug or other substance as a liquid or gas through an oral cavity or nasal cavity during an inhalation process. Such an inhaler is equipped with a container that accommodates an inhalable composition, and the composition may be sprayed from the container through a tube connected to the container and finally into the oral cavity or nasal cavity of a user through an intake. In addition to a liquid inhaler and a vapor inhaler, such an inhaler includes a powder inhaler that sprays fine powder of the composition as microparticles and an aerosol inhaler that supplies the composition by aerosolizing the composition.

A dry powder inhaler is intended to transfer a single-dose, metered dose of a pharmacological agent into the body and has been used primarily to treat patients with asthma and chronic obstructive pulmonary disease (COPD). Finely dried powders of 5 to 10 micrometers (µm) or less are used for efficient absorption of the agent into the body. In addition, a determined amount of drug may be filled into a hard capsule for quantitative delivery. Recently, the application fields of dry powder inhalers have expanded, and the scope has expanded to not only disposable or multi-use powder inhalers, but also powder inhalers that inhale medicinal substances, functional substances, nicotine, and other preference products.

The above description is information the inventor(s) acquired during the course of conceiving the present disclosure or already possessed at the time and is not necessarily art publicly known before the present application was filed.
Prior art document: Korea Patent Publication No. 10-1759972

### DISCLOSURE OF THE INVENTION

### TECHNICAL GOALS

Embodiments are to provide an inhaler that allows immediate inhalation through fastening of a dry powder cartridge without a separate capsule crushing device and has a function of removing impurities or particles that may clump together and cause coughing through an inertial dust collection principle.

The technical goals obtainable from the embodiments are not limited to the above-mentioned technical goals, and other unmentioned technical goals may be clearly understood from the following description by one of ordinary skill in the art to which the present disclosure pertains.

### TECHNICAL SOLUTIONS

An inhaler according to an embodiment for achieving the goals includes a main body that includes a first surface and a second surface facing the first surface and having a mouthpiece and has a channel formed inside in a longitudinal direction from the first surface toward the second surface, a cartridge coupled to the first surface of the main body, communicating with the channel, and containing an inhalable powder, and a plurality of baffles arranged in the channel, wherein, when a suction force is applied to the mouthpiece, the baffles may limit a particle size of the inhalable powder transferred to the mouthpiece.

According to an aspect, the baffles may include a first baffle member having one end fixed to an inner wall of the channel and another end extending toward a center line in the longitudinal direction of the channel and a second baffle member forming an angle with the other end of the first baffle member and further extending toward the center line, wherein the second baffle member may prevent particles that exceed a predetermined size of the inhalable powder from passing through the channel.

According to an aspect, the second baffle member may extend beyond the center line of the channel.

According to an aspect, the plurality of baffles may be spaced apart from each other within a range of 20 to 70 percent (%) of a length in the longitudinal direction of the main body.

According to an aspect, the channel may have a portion, which is adjacent to the second surface, formed in a spiral shape.

According to an aspect, the channel may have a cross-sectional area formed in a constant shape.

According to an aspect, the channel may be formed in a shape in which a cross-sectional area decreases as the channel approaches the second surface.

According to an aspect, the inhaler may further include a mesh net arranged between the main body and the cartridge, wherein the mesh net may limit the particle size of the inhalable powder flowing into the channel from the cartridge.

According to an aspect, a plurality of fastening members, which is magnetic, may be provided on each of the first surface of the main body and one side of the cartridge, and the fastening members of each of the main body and the cartridge may be detachably coupled to each other.

According to an aspect, a groove having a shape that matches a shape of an end portion adjacent to the first surface of the main body may be formed on one side of the cartridge, and an end portion of the main body is inserted into the groove in a fitting manner.

### EFFECTS OF THE INVENTION

According to an inhaler according to an embodiment, immediate inhalation is possible through fastening of a dry powder cartridge without a separate capsule crushing device, and impurities or particles that may clump together and cause coughing may be removed through an inertial dust collection principle.

The effects of the inhaler according to an embodiment are not limited to the above-mentioned effects, and other unmentioned effects can be clearly understood from the following description by one of ordinary skill in the art to which the present disclosure pertains.

### BRIEF DESCRIPTION OF DRAWINGS

FIG. 1 illustrates an inhaler according to an embodiment.
FIG. 2 illustrates an inhaler having a main body in which channels of different shapes are formed.
FIG. 3 illustrates a main body and a cartridge coupled in a magnetic fastening manner.
FIG. 4 illustrates a main body and a cartridge coupled in a fitting manner.

The accompanying drawings illustrate preferred embodiments of the present invention and are provided together with the detailed description for better understanding of the technical idea of the present invention. Therefore, the present invention should not be construed as being limited to the embodiments set forth in the drawings.

### BEST MODE FOR CARRYING OUT THE INVENTION

Hereinafter, embodiments are described in detail with reference to the illustrative drawings. Regarding the reference numerals assigned to the components in the drawings, it should be noted that the same components are designated by the same reference numerals, wherever possible, even though they are shown in different drawings. Furthermore, in the following description of the present embodiments, a detailed description of publicly known configurations or functions incorporated herein will be omitted when it is determined that the detailed description obscures the subject matters of the present embodiments.

In addition, the terms first, second, A, B, (a), and (b) may be used to describe components of the embodiments. These terms are used only for the purpose of discriminating one component from another component, and the nature, the sequences, or the orders of the components are not limited by the terms. When one component is described as being "connected", "coupled", or "attached" to another component, it should be understood that one component may be connected or attached directly to the other component, and an intervening component may also be "connected", "coupled", or "attached" to the components.

The same name may be used to describe an element included in the embodiments described above and an element having a common function. Unless otherwise mentioned, the descriptions of the embodiments may be applicable to the following embodiments and thus, duplicated descriptions will be omitted for conciseness.

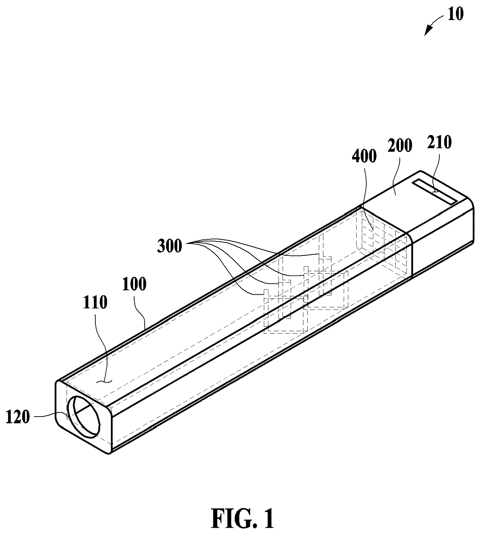

FIG. 1 is a diagram illustrating an inhaler 10 according to an embodiment.

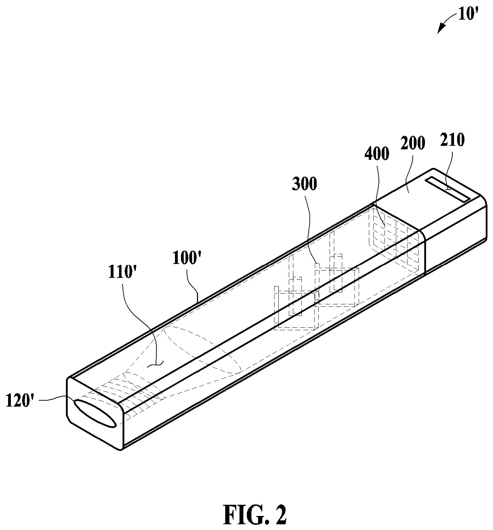

FIG. 2 illustrates an inhaler 10' having a main body 100' in which channels of different shapes are formed.

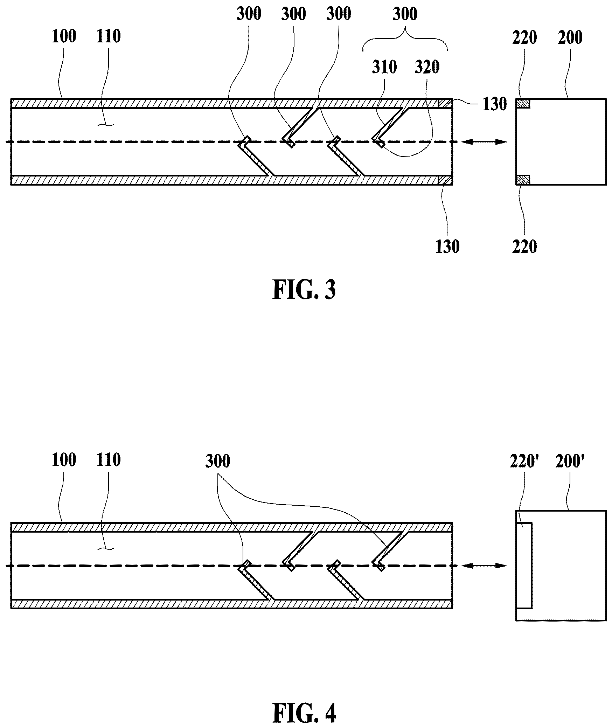

FIG. 3 illustrates a main body 100 and a cartridge 200 that are coupled together in a magnetic fastening manner.
FIG. 4 illustrates the main body 100 and a cartridge 200' coupled in a fitting manner.

Referring to FIG. 1, an inhaler 10 according to an embodiment may include the main body 100, the cartridge 200, and a baffle 300.

The main body 100 may include a first surface, a second surface opposite to the first surface, and a plurality of side surfaces surrounding the first surface and the second surface. The cartridge 200 may be coupled to the first surface. A mouthpiece 120 may be provided on the second surface. A channel 110 penetrating the first surface and the second surface may be formed inside the main body 100. The channel 110 may be a passage through which inhalable powder for transfer to the user may be transferred to the mouthpiece 120. Here, the inhalable powder may be transferred to the mouthpiece 120 along a longitudinal direction from the first surface to the second surface.

The channel 110 may be formed in a spiral shape with an end portion that is adjacent to the second surface and has a spiral shape. Accordingly, a vortex may be generated in the flow of inhalable powder.

Referring again to FIG. 1, the channel 110 may be formed in a shape with a constant cross-sectional area.

Referring to FIG. 2, a channel 110' may be formed in a shape in which the cross-sectional area decreases as the channel 110' approaches the second surface.

The cross-sectional area or cut-off size of the channel 110, 110' may be formed such that, for example, the flow rate of the inhalable powder may be transferred within 20 to 50 micrometers (µm) or 50 to 100 µm at 1.67 lpm.

In addition, the main body 100 may be provided with a flow distribution structure for controlling the degree of inhalation of the inhalable powder at the end portion adjacent to the second surface.

Furthermore, the main body 100 may include materials such as polylactic acid (PLA), acrylonitrile butadiene styrene copolymer (ABS), polycarbonate (PC), polypropylene (PP), etc. and may also include other biodegradable (eco-friendly) materials or metal.

The cartridge 200 may be coupled to the first surface of the main body 100. The cartridge 200 may contain an inhalable powder inside and may be connected internally with the channel 110 of the main body 100. The cartridge 200 may contain one or multiple doses of the inhalable powder. The inhalable powders may contain substances such as nicotine, nicotine salts, caffeine, taurine, vitamins, etc. In addition, the inhalable powder may include flavored particles, such as natural flavor particles, menthol, etc. The inhalable powder may be provided, for example, in dry powder form. Here, the dry powder used in the inhaler 10 may or may not have a carrier. The size of the inhalable powder may be approximately 5 to 10 µm and may include menthol particles larger than or equal to 20 µm. As described above, the inhalable powder may be transferred through the channel 110, thereby allowing a user to inhale the inhalable powder into the body.

In addition, the cartridge 200 may be detachably coupled to the first surface. Therefore, when the inhalable powder contained in the cartridge 200 is exhausted, a new cartridge 200 may be used as a replacement and mounted on the main body 100. Here, the cartridge 200 may be fastened by magnets or in a fitting manner. The method of fastening the cartridge 200 is described in more detail below with reference to FIGS. 3 and 4.

Furthermore, the inhaler 10 according to an embodiment may be a device that transfers the inhalable powder by utilizing an inertial force of particles, and when the particles of the transferred inhalable powder are larger than a certain size, the inhaler 10 may cause the user to cough. Accordingly, a plurality of baffles 300 may be provided in a zigzag structure as a filter structure to prevent particles or impurities larger than a certain size from being transferred into the body due to cohesion or aggregation between the particles.

The baffle 300 may be disposed in the channel 110.

When a suction force is applied to the mouthpiece 120, the inhalable powder contained in the cartridge 200 may pass through the channel 110 and may be transferred toward the mouthpiece 120. Here, the baffle 300 may limit a particle size of the inhalable powder transferred to the mouthpiece 120. For example, the baffle 300 may filter out particles larger than or equal to a predetermined size from the inhalable powder passing through the channel 110.

The plurality of baffles 300 may be provided, and each baffle 300 may be arranged to be spaced apart alternately on an inner wall of the channel 110 in the longitudinal direction, as shown in FIG. 1.

Referring to FIG. 3, the baffle 300 may include a first baffle member 310 and a second baffle member 320.

The first baffle member 310 may have one end fixed to the inner wall of the channel 110 and the other end extending toward a center line in the longitudinal direction of the channel 110. For example, the length of the first baffle member 310 may be at a level of 30, 40, 50, 60, 70, 80, or 90 percent (%) of a transverse length crossing the longitudinal direction of the channel 110.

The second baffle member 320 may extend further toward the center line while forming an angle with the other end of the first baffle member 310. The angle formed by the second baffle member 320 with the other end of the first baffle member 310 may be between 30 and 120 degrees. Preferably, the angle may be 90 degrees. The second baffle member 320 may extend further beyond the center line of the channel 110. This second baffle member 320 may not allow particles exceeding a predetermined size of the inhalable powder to pass through the channel 110.

In addition, the predetermined particle size filtered by the baffle 300 may vary depending on the length or shape of the first baffle member 310 and the second baffle member 320 or an arrangement interval of the first baffle member 310. In addition, an area in which the plurality of baffles 300 is arranged within the channel 110 may be at the level of 20, 30, 40, 50, 60, or 70 % of an entire length in the longitudinal direction of the channel 110.

As described above, by arranging the plurality of baffles in the channel 110 through which the inhalable powder passes, the inhaler 10 according to an embodiment may prevent particles or impurities larger than a certain size from being transferred into the body.

Referring again to FIGS. 1 and 2, the inhaler 10 according to an embodiment may further include a mesh net 400. The mesh net 400 may be disposed between the main body 100 and the cartridge 200. The mesh net 400 may limit the particle size of the inhalable powder flowing from the cartridge 200 into the channel 110. That is, the mesh net 400 may pre-filter the inhalable powder exceeding a predetermined size before the inhalable powder is filtered by the baffle 300. In addition, the mesh net may include the same material as the main body 100, such as PLA. Furthermore, the mesh net may include a chemically stable metal with no inhalation toxicity, such as stainless steel.

Referring to FIG. 3, the inhaler 10 according to an embodiment may be provided with a plurality of fastening members 220 in each of the main body 100 and the cartridge 200. A fastening member 220 may be disposed to be adjacent to the first surface of the main body 100. In addition, the fastening member 200 may be disposed on one side of the cartridge 200 in a position corresponding to the fastening member 220 disposed on the main body 100. The fastening member 220 may be provided as a magnetic structure, such as a magnet, for example. The fastening member 220 of the main body 100 and the fastening member 220 of the cartridge 200 may be provided with magnets of different polarities and may be detachably coupled to each other.

Referring to FIG. 4, the cartridge 200' of an inhaler according to another embodiment may have a groove 220' formed on one side. The groove 220' may be formed in a similar shape to align with an end portion adjacent to the first surface of the main body 100. Accordingly, the end portion of the main body 100 may be inserted and coupled to the groove 220' of the cartridge 200' in a fitting manner.

As described above, the inhaler 10 according to an embodiment may transfer the inhalable powder while selectively removing particles larger than a certain size by using the baffles 300 arranged in a zigzag structure, and the user may inhale the inhalable powder multiple times or once.

While the embodiments of the present disclosure have been described above with reference to specific components, and limited embodiments and drawings, the above descriptions are merely for better understanding of the present disclosure, and it will be apparent to one of ordinary skill in the art that various changes in form and details may be made in these embodiments without departing from the spirit and scope of the claims and their equivalents. For example, suitable results may be achieved if the described techniques are performed in a different order and/or if components in a described system, architecture, device, or circuit are combined in a different manner and/or replaced or supplemented by other components or their equivalents. Accordingly, the scope of the present disclosure is defined not by the detailed description, but by the claims and their equivalents, and all variations within the scope of the claims and their equivalents are to be construed as being included in the disclosure.

## Claims

1. An inhaler comprising:
a main body that comprises a first surface and a second surface facing the first surface and having a mouthpiece and has a channel formed inside in a longitudinal direction from the first surface toward the second surface;
a cartridge coupled to the first surface of the main body, communicating with the channel, and containing an inhalable powder; and
a plurality of baffles arranged in the channel;
wherein, when a suction force is applied to the mouthpiece, the baffles limit a particle size of the inhalable powder transferred to the mouthpiece.

2. The inhaler of claim 1, wherein
the baffles comprise:
a first baffle member having one end fixed to an inner wall of the channel and another end extending toward a center line in the longitudinal direction of the channel; and
a second baffle member forming an angle with the other end of the first baffle member and further extending toward the center line;
wherein the second baffle member prevents particles that exceed a predetermined size of the inhalable powder from passing through the channel.

3. The inhaler of claim 2, wherein
the second baffle member extends beyond the center line of the channel.

4. The inhaler of claim 1, wherein
the plurality of baffles is spaced apart from each other within a range of 20 to 70 percent (%) of a length in the longitudinal direction of the main body.

5. The inhaler of claim 1, wherein
the channel has a portion, which is adjacent to the second surface, formed in a spiral shape.

6. The inhaler of claim 1, wherein
the channel has a cross-sectional area formed in a constant shape.

7. The inhaler of claim 1, wherein
the channel is formed in a shape in which a cross-sectional area decreases as the channel approaches the second surface.

8. The inhaler of claim 1, further comprising:
a mesh net arranged between the main body and the cartridge,
wherein the mesh net limits the particle size of the inhalable powder flowing into the channel from the cartridge.

9. The inhaler of claim 1, wherein
a plurality of fastening members, which is magnetic, is provided on each of the first surface of the main body and one side of the cartridge, and
the fastening members of each of the main body and the cartridge are detachably coupled to each other.

10. The inhaler of claim 1, wherein
a groove having a shape that matches a shape of an end portion adjacent to the first surface of the main body is formed on one side of the cartridge, and
an end portion of the main body is inserted into the groove in a fitting manner.
